(19) Europäisches Patentamt / European Patent Office / Office européen des brevets

(11) **EP 2 349 558 B1**

(12) **FASCICULE DE BREVET EUROPÉEN**

(45) Date de publication et mention de la délivrance du brevet:
**01.06.2022 Bulletin 2022/22**

(21) Numéro de dépôt: **09760949.9**

(22) Date de dépôt: **22.10.2009**

(51) Classification Internationale des Brevets (IPC):
**B01J 23/887** (2006.01)    **C07C 319/06** (2006.01)
**B01J 23/58** (2006.01)    **B01J 23/78** (2006.01)

(52) Classification Coopérative des Brevets (CPC):
(C-Sets disponibles)
**B01J 23/8872; C07C 319/02;** B01J 23/58;
B01J 23/78                                     (Cont.)

(86) Numéro de dépôt international:
**PCT/FR2009/052035**

(87) Numéro de publication internationale:
**WO 2010/046607 (29.04.2010 Gazette 2010/17)**

(54) **PROCÉDÉ D'HYDROGÉNATION CATALYTIQUE DU SULFURE DE CARBONE EN MÉTHYLMERCAPTAN**

VERFAHREN ZUR KATALYTISCHEN HYDRIERUNG VON KOHLENSTOFFDISULFID ZU METHYLMERCAPTAN

PROCESS OF CATALYTIC HYDROGENATION OF CARBON DISULPHIDE TO METHYL MERCAPTAN

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **24.10.2008 FR 0857254**

(43) Date de publication de la demande:
**03.08.2011 Bulletin 2011/31**

(73) Titulaire: **Arkema France 92700 Colombes (FR)**

(72) Inventeurs:
• **BARRE, Patrice**
**64140 Lons (FR)**
• **FREMY, Georges**
**64390 Sauveterre-de-Béarn (FR)**
• **LOZOWSKI, Andre**
**64230 Lescar (FR)**

(74) Mandataire: **Arkema Patent
Arkema France
DRD-DPI
420, rue d'Estienne d'Orves
92705 Colombes Cedex (FR)**

(56) Documents cités:
**EP-A- 0 796 656    FR-A- 2 419 281
FR-A- 2 864 102    US-A- 3 488 739
US-A- 3 880 933**

• **None**

(52) Classification Coopérative des Brevets (CPC):
(Cont.)

C-Sets
**C07C 319/02**

**Description**

La présente invention concerne le procédé de préparation de méthylmercaptan par hydrogénation catalytique de disulfure de carbone.

La réaction d'hydrogénation du $CS_2$ en méthylmercaptan peut être schématisée comme suit:

$$CS_2 + 3H_2 \rightarrow CH_3SH + H_2S$$

Les sous-produits de cette réaction sont le méthane, par hydrogénolyse du $CH_3SH$, et le sulfure de diméthyle (DMS), par condensation de deux molécules de $CH_3SH$.

Cette réaction d'hydrogénation, bien que connue depuis des décennies, ne semble pas avoir fait l'objet de recherches intensives, si l'on en juge par les quelques rares publications brevets disponibles aujourd'hui.

Par exemple, le brevet US 3 488 739 (The Sun Oil Company, 1970) décrit un procédé de préparation de méthylmercaptan et de sulfure de diméthyle à partir de $CS_2$ et d'hydrogène, utilisant un catalyseur d'hydrogénation (Aero-HDS-3A) qui est un mélange d'oxydes de nickel et de molybdène sur alumine.

À 204°C, la conversion en $CS_2$ est d'environ 100% et les sélectivités molaires sont de 33% en $CH_3SH$ et de 67% en DMS, avec des traces de méthane.

À 177°C, la conversion en $CS_2$ chute à 76% molaires et le rapport molaire des sélectivités $CH_3SH$/DMS est de 50/50.

Le brevet mentionne également la possibilité de recycler le $CH_3SH$ ou le DMS afin de favoriser la formation de l'un ou l'autre de ces composés. Toutefois, un tel recyclage entraîne la formation de quantités non négligeables de méthane.

Le brevet US 3 880 933 (Phillips, 1975) décrit un procédé d'hydrogénation catalytique de $CS_2$ en méthylmercaptan, en présence de sulfure d'hydrogène ($H_2S$). Le catalyseur utilisé est un mélange d'oxydes de cobalt et de molybdène sur alumine (Aero-HDS-2).

La température réactionnelle mentionnée est comprise entre 230°C et 260°C, la pression réactionnelle entre 11,9 et 12,6 bars et le ratio molaire $H_2$/$CS_2$ entre 2,75 et 3,5 (3 étant le ratio stœchiométrique pour la production de $CH_3SH$).

En utilisant une quantité raisonnable de $H_2S$ présent au départ (ratio molaire $H_2S$/$CS_2$ = 3), le meilleur résultat donne un taux de conversion en $CS_2$ de 69% et une sélectivité molaire en $CS_2$ de 65% et de 35% pour le DMS, calculs effectués à partir des compositions molaires indiqués dans le brevet.

En 1985 et 1989, trois brevets de Mobil Oil (US 4 543 434, US 4 822 938, US 4 864 074) décrivent des procédés de conversion de méthane en hydrocarbures de plus haut poids moléculaires, en utilisant des composés intermédiaires contenant du soufre.

Dans ces brevets, le méthane est converti en $CS_2$/$H_2S$ par oxydation au soufre (similaire au procédé Folkins) puis, sous l'action d'hydrogène, le $CS_2$ est converti en hydrocarbures supérieurs. Sans que cela ne soit exemplifié, la description de ces brevets mentionne que le $CS_2$ est tout d'abord converti en $CH_3SH$ et que ce dernier est l'intermédiaire final vers les hydrocarbures. Il est également mentionné que le DMS peut être coproduit durant la réaction entre le $CS_2$ et l'hydrogène.

La demande internationale de brevet WO 2004/043883 (Georgia Pacific) décrit quant à elle un procédé de conversion catalytique de disulfure de carbone en méthylmercaptan, en présence d'hydrogène. Le catalyseur peut être $V_2O_5$, $Re_2O_7$ ou MnO, supporté sur un substrat de $CeO_2$, $ZrO_2$, $TiO_2$, $Nb_2O_5$, $Al_2O_3$, $SiO_2$, $Ta_2O_5$, $SnO_2$, ou des mélanges de ceux-ci.

Il est mentionné un taux de conversion élevé en $CS_2$, ainsi qu'une sélectivité élevée en $CH_3SH$, mais également la formation de sous-produits, tels que $CH_4$, $C_2H_6$, etc., et également DMS. En outre, cette demande ne fournit aucun exemple concret, et aucune valeur précise de conversion ni de sélectivité n'est présentée.

Cette revue de l'art antérieur permet de constater d'une part le faible nombre de publications concernant la réaction d'hydrogénation catalytique de $CS_2$ en méthylmercaptan, et d'autre part le manque d'information pour conduire cette réaction avec des taux de conversion élevés, ainsi que des sélectivités élevées en méthylmercaptan.

Les données existantes montrent par exemple que des sélectivités en $CH_3SH$ supérieures à 65%, dans des conditions acceptables sur le plan industriel, n'ont jamais été obtenues.

Aussi, un objectif de la présente invention consiste à proposer une réaction d'hydrogénation de disulfure de carbone en méthylmercaptan, de mise en œuvre facile et économique sur le plan industriel, en particulier avec de faibles coûts variables.

Un autre objectif de l'invention est de proposer une telle réaction ne produisant que peu ou pas de sous-produit, c'est-à-dire fournissant des taux de conversion élevés, ainsi qu'une sélectivité élevée en produit hydrogéné, à savoir le méthylmercaptan, autrement dit un procédé présentant des performances accrues en termes de rendement, conversion, sélectivité et productivité.

L'invention a également pour objectif de proposer un procédé de conversion de $CS_2$ en $CH_3SH$ produisant de

faibles émissions de dioxyde de carbone ($CO_2$).

D'autres objectifs encore apparaîtront au cours de l'exposé de qui suit.

Les inventeurs ont maintenant découvert que ces objectifs peuvent être atteints en totalité ou en partie grâce au procédé de l'invention qui suit, et notamment grâce à un type de catalyseur spécifique.

Ainsi, la présente invention utilise un catalyseur d'hydrogénation comprenant des métaux dopés par au moins un hydroxyde ou oxyde de potassium.

Le métal présent dans le catalyseur de l'invention est choisi dans les combinaisons Co/Mo, Ni/Mo, Ni/W, W/Mo, les combinaisons de nickel et de molybdène étant préférées.

Les métaux présents dans le catalyseur de l'invention se présentent le plus souvent sous forme d'oxydes, et sont disponibles dans le commerce ou bien facilement préparés à partir de modes opératoires connus de l'homme du métier.

Un catalyseur tout particulièrement préféré est le catalyseur commercialisé par la société Axens sous la dénomination HR 448, qui est une combinaison d'oxyde de nickel (NiO) et d'oxyde de molybdène ($MoO_3$), supportée sur alumine de haute pureté. Ce catalyseur peut être utilisé en association avec d'autres catalyseurs de type Ni/Mo et/ou Co/Mo. En outre, ce catalyseur, comme tout catalyseur de l'invention, peut être pré-sulfuré, comme indiqué ci-après.

Les métaux présents dans le catalyseur peuvent également se présenter directement sous la forme de sulfures métalliques. Ces sulfures métalliques peuvent aussi être obtenus à partir des oxydes correspondants selon toute méthode connue de l'homme du métier, par exemple comme décrit par van Venrooy dans le brevet précité US 3 488 739.

Le catalyseur est avantageusement supporté, de manière conventionnelle, sur tout type de support généralement utilisé dans ce domaine, et par exemple sur un support choisi parmi alumine, silice, dioxyde de titane ($TiO_2$), zéolites, charbon, zircone, magnésie (MgO), les argiles, les hydrotalcites et autres, ainsi que les mélanges de deux ou plusieurs d'entre eux.

Le catalyseur se caractérise par le fait qu'il est dopé, avant utilisation, par au moins un oxyde et/ou hydroxyde de potassium, la quantité d'agent de dopage étant généralement comprise entre 8% et 14% en poids d'oxyde et/ou d'hydroxyde de potassium par rapport au poids total du catalyseur.

Le dopage indiqué ci-dessus peut être effectué selon toute méthode connue de l'homme du métier, et par exemple par imprégnation à sec d'au moins un hydroxyde et/ou oxyde de potassium.

Selon un mode de réalisation préféré de l'invention, le dopage est réalisé par imprégnation à sec d'une solution d'hydroxyde de potassium (KOH) et/ou d'oxyde de potassium ($K_2O$).

Le catalyseur (métal dopé par un hydroxyde et/ou oxyde de potassium) s'est montré particulièrement efficace dans les réactions d'hydrogénation, en particulier d'hydrogénation de composés soufrés, plus particulièrement les composés soufrés porteurs d'au moins un groupe C=S. Le catalyseur est plus particulièrement efficace pour la réaction d'hydrogénation catalytique de $CS_2$ en méthylmercaptan, pour laquelle des conversions de $CS_2$ et des sélectivités en $CH_3SH$ élevées, notamment de l'ordre de 100% ont été observées.

Le catalyseur, notamment lorsqu'il est composé d'oxyde(s) métallique(s) est avantageusement pré-traité (présulfuré), avant d'être utilisé pour la réaction d'hydrogénation catalytique du $CS_2$ en $CH_3SH$. Le pré-traitement consiste à transformer le(s) oxyde(s) métallique(s) en sulfures correspondants, par exemple selon les indications fournies par van Venrooy (US 3 488 739).

A titre d'exemple illustratif et non limitatif, le catalyseur peut être pré-traité (pré-sulfuré) par un mélange hydrogène/sulfure d'hydrogène ($H_2/H_2S$), en proportion par exemple 20/80 molaire (ou volumique). Le pré-traitement est effectué à une température comprise entre 200°C et 400°C, de préférence à une température d'environ 300°C, pendant une durée suffisante pour assurer une conversion supérieure à 80%, de préférence supérieure à 90%, de préférence encore une conversion totale, des oxydes métalliques en sulfures correspondants, soit une durée d'environ 4 heures pour une conversion totale.

Le catalyseur est alors prêt pour être directement engagé dans une réaction catalytique d'hydrogénation.

La présente invention concerne un procédé d'hydrogénation catalytique du disulfure de carbone selon la revendication 1, ledit procédé étant caractérisé par le fait que le catalyseur d'hydrogénation est un catalyseur tel que celui défini précédemment.

Plus particulièrement, le procédé selon l'invention comprend au moins les étapes suivantes :

a) mise en contact du disulfure de carbone, avec de l'hydrogène, en présence d'au moins un catalyseur tel que défini précédemment, ayant éventuellement subi le pré-traitement de conversion des oxydes métalliques en sulfures métalliques, comme décrit ci-dessus ;

b) conduite de la réaction sous pression d'hydrogène à une température comprise entre 100°C et 400°C, de préférence comprise entre 200°C et 300°C ; et

c) récupération du produit d'hydrogénation, le méthylmercaptan, avec séparation du $H_2S$ formé.

Dans le cas de la réaction d'hydrogénation catalytique de disulfure de carbone en méthylmercaptan, le ratio

molaire $H_2/CS_2$ peut varier.

Les divers essais réalisés par les inventeurs montrent que plus la quantité d'hydrogène est importante, par rapport à la quantité de $CS_2$ introduit, plus la productivité par rapport à un volume donné de catalyseur diminue. Aussi, on travaille avec un ratio $H_2/CS_2$ compris entre 1:1 et 10:1, de préférence encore entre 2:1 et 9:1. Des ratios environ égaux à 3:1, 6:1 et 9:1 ont fourni de très bons résultats, les meilleurs résultats étant obtenus pour un ratio $H_2/CS_2$ compris entre 3:1 et 6:1.

La quantité de catalyseur peut également varier dans de très grandes proportions. Celle-ci est en effet directement reliée à la productivité souhaitée. Le plus souvent, on exprime la productivité en quantité de méthylmercaptan produite par unité de temps et par unité de volume de catalyseur (ou unité de masse de catalyseur).

En règle générale, la quantité de catalyseur est fixée en fonction de la nature et de la quantité du composé à hydrogéné. Ainsi, une quantité de 0,5 à 6 kg/heure/Litre de catalyseur de composé à hydrogéner, en particulier de disulfure de carbone, est suffisante, voire optimale pour une réaction d'hydrogénation en méthylmercaptan.

Il est également possible d'utiliser un composé inerte vis-à-vis du catalyseur afin de diluer l'exotherme de la réaction. Un tel composé inerte est bien connu de l'homme du métier, spécialiste des réactions d'hydrogénation catalytique, et peut être par exemple du carborundum (carbure de silicium).

Comme indiqué plus haut, la réaction d'hydrogénation est conduite sous pression d'hydrogène. La pression nécessaire à la réaction peut, ici encore, varier dans de grandes proportions. Elle est généralement comprise entre la pression atmosphérique et 100 bars (environ 10 Mpa), de préférence entre la pression atmosphérique 50 bars (environ 5 Mpa), et de préférence encore entre la pression atmosphérique et 30 bars (3 Mpa). Selon un aspect tout à fait préféré, la pression d'hydrogène est comprise entre 3 bars (0,3 Mpa) et 15 bars (1,5 Mpa).

La pression d'hydrogène a, entre autres, un effet important sur le temps de contact entre les réactifs et le catalyseur. Une pression élevée, donc un temps de contact important, peut s'avérer bénéfique pour la conversion du substrat (ici le $CS_2$). Cependant des temps de contact trop importants peuvent également favoriser des réactions parasites ; c'est pourquoi, un compromis est à trouver pour des performances optimales.

La réaction d'hydrogénation catalytique de disulfure de carbone en méthylmercaptan peut être conduite en présence de sulfure d'hydrogène, qui est lui-même formé au cours de la réaction. Une quantité de sulfure d'hydrogène pouvant aller jusqu'à 10 $H_2S$ pour 1 $CS_2$ en mole n'a pas d'influence sur la productivité, le rendement, le taux de conversion, et la sélectivité de la réaction.

La température réactionnelle est généralement comprise entre 100°C et 400°C, de préférence entre 200°C et 300°C, ainsi qu'il a été mentionné précédemment. Il a été observé qu'une température trop élevée a un effet néfaste sur la sélectivité, avec formation de sous-produits non désirés dans le cadre de la présente invention, tels que méthane et sulfure de diméthyle. En revanche, une température trop basse entraîne une chute de conversion du produit à hydrogéner, typiquement le disulfure de carbone.

Le catalyseur peut être utilisé selon toute méthode connue dans le domaine, par exemple en lit fixe, fluide, circulant ou bouillonnant. Dans les exemples illustrant l'invention, le catalyseur a été utilisé en lit fixe.

De même, si tous les types de réacteurs d'hydrogénation peuvent être envisagés pour la réaction d'hydrogénation selon la présente invention, on préfère les réacteurs qui permettent de bons échanges thermiques, afin de pouvoir évacuer le plus efficacement possible la chaleur de la réaction.

La durée de la réaction varie selon la nature et la quantité du produit à hydrogéner, la quantité d'hydrogène et la nature et la quantité de catalyseur utilisé. Cette durée est généralement très courte, par exemple de l'ordre de la seconde, ce qui rend le procédé de l'invention tout à fait adapté pour une conduite de la réaction en continu.

La réaction est avantageusement conduite sans solvant, sans eau. En outre, si nécessaire ou si on l'on souhaite, elle peut être conduite en présence d'un gaz inerte, tel que azote, argon, et autres.

Le méthylmercaptan obtenu à l'issue de l'hydrogénation de disulfure de carbone, peut être séparé du brut réactionnel selon tout moyen classique connu de l'homme du métier, par exemple par condensation, par refroidissement, éventuellement par augmentation de pression.

Le procédé de l'invention, avantageusement conduit en continu, permet notamment l'effectuer l'hydrogénation de disulfure de carbone avec une grande productivité, un taux de conversion élevé en disulfure de carbone, et une grande sélectivité en méthylmercaptan, c'est-à-dire une formation exclusive, ou quasi-exclusive de méthylmercaptan, sans observer la formation des sous-produits indésirables, habituellement formés avec les procédés décrits dans l'art antérieur.

Les caractéristiques exposés ci-dessus rendent le procédé particulièrement adapté à une production industrielle de $CH_3SH$ de haute pureté par hydrogénation catalytique de disulfure de carbone, et notamment la production de $CH_3SH$ de pureté supérieure à 99,9%, avec des impuretés en quantités négligeables, inférieures aux seuils de détection, et notamment inférieures à 1000 ppm.

La présente invention est maintenant illustrée au moyen des exemples qui suivent, qui ne présentent aucun caractère limitatif, et qui ne peuvent être par conséquent compris comme susceptibles de restreindre la portée de l'invention telle que revendiquée.

Pour chacun des exemples, les produits réactionnels et les produits n'ayant pas réagi sont vaporisés et analysés en ligne par chromatographie en phase gazeuse avec une colonne capillaire apolaire et un catharomètre comme détecteur, après calibrage pour chaque produit avec des échantillons purs.

Les taux de conversion, sélectivité et productivité sont calculés de la manière suivante (pour chaque calcul, le nombre de moles de produit $i$ est égal au débit molaire du produit $i$ multiplié par unité de temps.) :

• **_Taux de conversion de CS$_2$ (%C) :_**

Si $n_{0CS_2}$ est le nombre de moles initial de CS$_2$ et $n_{CS_2}$ résiduel, le nombre de moles de CS$_2$ n'ayant pas réagi, le taux de conversion de CS$_2$ (%C) peut être calculé selon l'équation suivante :

$$\%C \;=\; \frac{n_{0\,CS_2} \,-\, n_{CS_2\ \text{résiduel}}}{n_{0\,CS_2}} \times 100$$

• **_Sélectivité molaire en CH$_3$SH (%S$_{CH3SH}$) :_**

Si $n_{CH3SH}$ est le nombre de moles de CH$_3$SH produites au cours de la réaction, la sélectivité molaire en CH$_3$SH (%S$_{CH3SH}$) peut être calculée selon l'équation suivante :

$$\%S_{CH_3SH} \;=\; \frac{n_{CH_3SH}}{n_{0\,CS_2} - n_{CS_2\ \text{résiduel}}} \times 100$$

• **_Sélectivité molaire en CH$_4$ (%S$_{CH4}$) :_**

La sélectivité molaire en méthane (%S$_{CH4}$) peut également être calculée à partir de l'équation suivante :

$$\%S_{CH_4} \;=\; \frac{n_{CH_4}}{n_{0\,CS_2} - n_{CS_2\ \text{résiduel}}} \times 100$$

• **_Sélectivité molaire en sulfure de diméthyle (%S$_{DMS}$) :_**

De même, la sélectivité molaire en DMS (%S$_{DMS}$) est calculée en utilisant l'équation suivante :

$$\%S_{DMS} \;=\; \frac{2 \times n_{DMS}}{n_{0\,CS_2} - n_{CS_2\ \text{résiduel}}} \times 100$$

• **_Productivité en CH$_3$SH (%P$_{CH3SH}$) :_**

La productivité en méthylmercaptan est alors calculée à partir de l'équation suivante (où $M_{CH3SH}$ et $M_{CS_2}$ représentent respectivement la masse molaire de CH$_3$SH et la masse molaire de CS$_2$) :

$$P_{CH3SH} = \text{débit massique de CS}_2 \text{ x \%C x \%S}_{CH3SH} \text{ x } \frac{M_{CH3SH}}{M_{CS2}}$$

où le débit massique de CS$_2$ est exprimé en tonnes/jour/m$^3$ de catalyseur.

### Exemple 1 : Préparation des catalyseurs

Les essais sont réalisés sur quatre catalyseurs, un catalyseur non dopé (Cata 1, exemple comparatif), et trois catalyseurs dopés avec différentes quantités d'oxyde de potassium (Cata 2 : 5,8% ; Cata 3 : 11,6% et Cata 4 : 17,4% en poids). Les essais réalisés sur les catalyseurs Cata 2 et Cata 4 ne sont pas conforme à l'invention.

Cata 1 est le catalyseur commercial HR 448 de la société Axens (3,3% d'oxyde de nickel et 16,5% de trioxyde de molybdène sur support alumine). Les trois autres catalyseurs sont préparés selon le mode opératoire suivant.

Pour chaque catalyseur Cata 2, Cata 3 et Cata 4, 100 mL (79 g) de catalyseur HR 448 (Axens), préalablement séché à 150°C pendant une nuit, sont introduits dans un évaporateur rotatif.

Parallèlement, une solution d'hydroxyde de potassium est préparée en dissolvant 5,80 g d'hydroxyde de potassium (KOH) dans 38,6 mL d'eau déminéralisée. Ce volume correspond au volume poreux des 79 g de HR448 (déterminé au préalable avec de l'eau déminéralisée sur un échantillon représentatif du lot de catalyseur utilisé). Pour les fortes concentrations (17,4% $K_2O$), l'hydroxyde de potassium est dissous dans l'eau chaude.

La solution est alors introduite lentement et en continu, dans l'évaporateur rotatif (un léger vide permet à la solution d'être introduite par différence de pression). Tout au long de l'introduction, le récipient contenant le catalyseur est mis en faible rotation, afin de favoriser un imprégnation homogène.

L'opération d'imprégnation est réalisée est maintenue à température ambiante, afin d'éviter toute évaporation d'eau avant la fin de l'introduction de la solution.

À la fin de l'introduction, l'eau est éliminée sous vide et chauffage à 100°C. Après ce séchage "*in situ*", le catalyseur Cata 2 imprégné est calciné à 500°C pendant 2 heures et est alors prêt à être utilisé, après refroidissement.

Selon un mode opératoire similaire, Cata 3 est obtenu à partir de 12,35 g de KOH pour conduire à un catalyseur dopé à 11,6% de $K_2O$ ; Cata 4 est obtenu de manière similaire à partir de 19,82 g de KOH, pour conduire à un catalyseur dopé à 17,4% de $K_2O$.

Les catalyseurs (contenant des oxydes métalliques) sont soumis au pré-traitement suivant : 30 mL de catalyseur sont introduits dans un réacteur tubulaire. Le catalyseur est alors mis en contact avec un mélange de $H_2S$ (8 litres Normaux/heure) et de $H_2$ (2 litres Normaux/heure) à 400°C pendant 4 heures. La pression dans le réacteur tubulaire est fixée à une valeur identique à celle de la réaction d'hydrogénation, entre 3 bars (0,3 Mpa) et 15 bars (1,5 Mpa), par exemple 3 bars (0,3 Mpa).

Les catalyseurs sulfurés sont récupérés et utilisés directement dans les réactions d'hydrogénation exemplifiées ci-dessous.

## Réactions d'hydrogénation de $CS_2$ en $CH_3SH$

### Exemple 2 : Étude de l'influence du dopage

Les catalyseurs obtenus à l'exemple 1 (Cata 2, Cata 3 et Cata 4) sont directement utilisés après le pré-traitement au soufre. Cata 1 (catalyseur non dopé et ayant également subi le pré-traitement au soufre) est utilisé à titre d'exemple comparatif. La quantité de catalyseur utilisée est généralement de 30 mL, certains essais ayant été effectués avec 6 mL et 10 mL de catalyseur. En outre, dans certains essais réalisés avec 6 mL de catalyseur, ledit catalyseur a été dilué à 30 mL avec un composé inerte (carborundum).

Les débits de $H_2$ et de $H_2S$ sont ajustés afin de correspondre au ratio molaire requis de $CS_2$, par exemple pour un ratio molaire $H_2S/CS_2$ égal à 2 et un ratio molaire $H_2/CS_2$ égal à 6, le débit de $CS_2$ est égal à 11,3 g/h (149 mmol/h, soit 3,3 litres gaz Normaux/h).

Différents tests sont effectués avec un ratio molaire $H_2/CS_2$ compris entre 3 et 9. Le Tableau 1 suivant donne, à titre d'exemples illustratifs, le débit de $CS_2$ à appliquer en fonction du ratio $H_2/CS_2$ choisi.

-- Tableau 1 --

| Ratio $H_2$/ $CS_2$ | Débit de $CS_2$ |
|---|---|
| 3 | 17 g/h (567 g/h/litre de catalyseur) |
| 6 | 11,3 g/h (377 g/h/litre de catalyseur) |
| 9 | 7,5 g/h (250 g/h/litre de catalyseur) |
| 30 | 2 g/h (67 g/h/litre de catalyseur) |

Le débit de $CS_2$ est modifié avec le ratio molaire $H_2/CS_2$, afin de maintenir un temps de séjour toujours identique. Le débit de $H_2$ peut être déduit aisément lorsque le ratio molaire est connu Ainsi, dans l'exemple où le ratio molaire $H_2/CS_2$ est de 6 (voir ci-dessus), le débit de $CS_2$ est de 11,3 g/h soit 3,3 litres gazeux Normaux/h, le débit d'hydrogène $H_2$ est égal à 6 fois ce débit de $CS_2$ soit 20 litres Normaux par heure. De même, le débit de $H_2S$ peut être déduit aisément lorsque le ratio molaire est connu. Ainsi, de la même façon qu'avec l'hydrogène dans l'exemple ci-dessus, un ratio molaire $H_2S/CS_2$ de 2 signifie que le débit d'$H_2S$ est égal à 2 fois le débit volumique gazeux du $CS_2$, soit, ici, 6,6 litres normaux par heure (2 fois 3,3).

En même temps, le réacteur tubulaire est refroidi à la température désirée de la réaction d'hydrogénation, c'est-à-dire entre 200°C et 300°C, par exemple 250°C. Le $CS_2$ est introduit seulement lorsque tous les débits et la température sont stabilisés.

À la sortie du réacteur, tous les produits de la réaction et les produits n'ayant pas réagi sont vaporisés et analysés en ligne par chromatographie en phase gazeuse.

Pour les différents essais conduits avec les catalyseurs dopés (Cata 2, Cata 3 et Cata 4), aucune formation de méthane, ni de sulfure de diméthyle n'est observée. Les gaz issus du réacteur ne contiennent également pas de $CS_2$ n'ayant pas réagi. Il peut donc être conclu que le taux de conversion en $CS_2$ est de 100% et la sélectivité en $CH_3SH$ est de 100%.

Les données opératoires et analytiques des différents essais sont rassemblées dans le Tableau 2 suivant :

-- Tableau 2 --

| Conditions opératoires générales : | | | | |
|---|---|---|---|---|
| • Volume de catalyseur : 30 mL<br>• Température réactionnelle : 25 0°C<br>• Pression réactionnelle (Mpa) : 0,3 3<br>• Ratio molaire $H_2/CS_2$ : 6<br>• Ratio molaire $H_2S/CS_2$ : 2 | | | | |
| **N° de l'essai** | **2.1** | **2.2** | **2.3** | **2.4** |
| *Catalyseur* | Cata 1 | Cata 2 | Cata 3 | Cata 4 |
| *Débit $CS_2$ (g/h)* | 10,0 | 11,3 | 11,3 | 11,3 |
| *Conversion $CS_2$ (%)* | 100 | 100 | 100 | 55 |
| *Sélectivité $CH_3SH$ (%)* | 29 | 71 | 100 | 100 |
| *Sélectivité $CH_4$ (%)* | 37 | 21 | 0,0 | 0,0 |
| *Sélectivité DMS (%)* | 34 | 7 | 0,0 | 0,0 |

Les résultats ci-dessus montrent l'influence du dopage du catalyseur d'hydrogénation sur la sélectivité en méthylmercaptan. Bien qu'une absence de dopage permette une conversion totale de $CS_2$, la réaction d'hydrogénation n'est pas du tout sélective : méthylmercaptan, méthane et sulfure de diméthyle sont formés en quantités sensiblement égales.

En revanche, le dopage du catalyseur permet d'augmenter considérablement la sélectivité en méthylmercaptan, jusqu'à 100% avec 11,6% de dopant $K_2O$ et au-delà. On constate également qu'une augmentation du taux de dopage entraîne une perte de conversion du disulfure de carbone.

### Exemple 3 : Étude de l'influence de la température

Des essais d'hydrogénation catalytique de disulfure de carbone en méthylmercaptan sont réalisés, selon le mode opératoire décrit à l'exemple 2 ci-dessus, à différentes températures (200°C, 250°C, et 300°C).

Les données opératoires et analytiques de ces trois essais sont rassemblées dans le Tableau 3 suivant :

-- Tableau 3 --

| Conditions opératoires générales : | | | |
|---|---|---|---|
| • Catalyseur : Cata 3<br>• Volume de catalyseur : 30 mL<br>• Débit $CS_2$ (g/h) : 11,3<br>• Pression réactionnelle (Mpa) : 0,3<br>• Ratio molaire $H_2/CS_2$ : 6<br>• Ratio molaire $H_2S/CS_2$ : 2 | | | |
| **N° de l'essai** | **3.1** | **3.2\*** | **3.3** |
| *Température réactionnelle (°C)* | 200 | 250 | 300 |
| *Conversion $CS_2$ (%)* | 31,6 | 100 | 100 |

(suite)

| N° de l'essai | | 3.1 | 3.2* | 3.3 |
|---|---|---|---|---|
| Sélectivité CH₃SH (%) | | 100 | 100 | 70,6 |
| Sélectivité CH₄ (%) | | 0,0 | 0,0 | 29,4 |
| Sélectivité DMS (%) | | 0,0 | 0,0 | 0,0 |
| * correspond à l'essai 2.3. | | | | |

Les résultats de ces essais montrent que la température n'a que peu d'influence sur la réaction d'hydrogénation, notamment concernant la sélectivité en méthylmercaptan. Même à une température réactionnelle de 30°C, peu de méthane est formé, et aucune trace de DMS n'a pu être mise en évidence.

**Exemple 4** : **Étude de l'influence du ratio $H_2/CS_2$**

De manière similaire à l'exemple 2 ci-dessus, différents essais d'hydrogénation catalytique de disulfure de carbone en méthylmercaptan sont réalisés, en faisant cette fois-ci varier la quantité d'hydrogène introduite. Le ratio molaire $H_2/CS_2$ a été fixé respectivement à 3, 6 et 9.

Les données opératoires et analytiques de ces trois essais sont rassemblées dans le Tableau 4 suivant :

**-- Tableau 4 --**

| Conditions opératoires générales : | | | |
|---|---|---|---|
| • Catalyseur : Cata 3 | | | |
| • Volume de catalyseur : 30 mL | | | |
| • Débit CS₂ (g/h) : 11,3 | | | |
| • Pression réactionnelle (Mpa) : 0,3 | | | |
| • Température réactionnelle (°C) : 250°C | | | |
| • Ratio molaire H₂S/CS₂ : 2 | | | |
| N° de l'essai | 4.1 | 4.2* | 4.3 |
| Ratio H₂/CS₂ | 3 | 6 | 9 |
| Conversion CS₂ (%) | 83,6 | 100 | 100 |
| Sélectivité CH₃SH (%) | 100 | 100 | 100 |
| Sélectivité CH₄ (%) | 0,0 | 0,0 | 0,0 |
| Sélectivité DMS (%) | 0,0 | 0,0 | 0,0 |
| * correspond à l'essai 2.3 | | | |

Les résultats ci-dessus montrent que, avec un ratio $H_2/CS_2$ égal à 3, la quantité d'hydrogène est insuffisante pour convertir la totalité du disulfure de carbone. En revanche, pour tous les ratios de 3, 6 et 9, la sélectivité en méthylmercaptan est de 100%, et ce, grâce au dopage du catalyseur, conformément à l'invention.

**Exemple 5** : **Étude de l'influence de la pression réactionnelle**

De manière similaire à l'exemple 2 ci-dessus, différents essais d'hydrogénation catalytique de disulfure de carbone en méthylmercaptan sont réalisés, en faisant cette fois-ci varier la pression appliquée au milieu réactionnel, en fonction de la température. Les pressions testées sont de 0,3 Mpa et 1,5 Mpa, les températures réactionnelles de 200°C et 250°C.

Les données opératoires et analytiques de ces essais sont rassemblées dans le Tableau 5 suivant :

**-- Tableau 5 --**

| Conditions opératoires générales : | |
|---|---|
| • Catalyseur : Cata 3 | |

(suite)

| Conditions opératoires générales : | | | | |
|---|---|---|---|---|
| • Volume de catalyseur :      30 mL | | | | |
| • Débit $CS_2$ (g/h) :      11,3 | | | | |
| • Ratio molaire $H_2/CS_2$ :      6 | | | | |
| • Ratio molaire $H_2S/CS_2$ :      2 | | | | |
| *N° de l'essai* | *5.1\** | *5.2* | *5.3\*\** | *5.4* |
| *Pression réactionnelle (Mpa)* | 0,3 | 1,5 | 0,3 | 1,5 |
| *Température réactionnelle (°C)* | 200 | 200 | 250 | 250 |
| *Conversion $CS_2$ (%)* | 31,6 | 100 | 100 | 100 |
| *Sélectivité $CH_3SH$ (%)* | 100 | 100 | 100 | 82,9 |
| *Sélectivité $CH_4$ (%)* | 0,0 | 0,0 | 0,0 | 17,1 |
| *Sélectivité DMS (%)* | 0,0 | 0,0 | 0,0 | 0,0 |
| \* correspond à l'essai 3.1<br>\*\* correspond à l'essai 2.3 | | | | |

Les résultats ci-dessus montrent qu'à température et pression élevées une conversion totale en $CS_2$ est conservée, avec toutefois une formation de méthane. Tous les essais montrent bien l'absence de formation de DMS dans chacun des cas.

**Revendications**

**1.** Procédé d'hydrogénation catalytique qui est une réaction d'hydrogénation catalytique du disulfure de carbone ($CS_2$) en méthylmercaptan, dans lequel le catalyseur d'hydrogénation est un catalyseur comprenant les combinaisons de métaux Co/Mo, Ni/Mo, Ni/W ou W/Mo ;

lesdits métaux étant dopés par l'hydroxyde de potassium ou l'oxyde de potassium, et la quantité d'hydroxyde de potassium ou d'oxyde de potassium par rapport au poids total du catalyseur est comprise entre 8% et 14% en poids ; et
dans lequel le ratio molaire $H_2/CS_2$ est compris entre 1:1 et 10:1.

**2.** Procédé selon la revendication 1, dans lequel la combinaison de deux métaux est une combinaison de nickel et de molybdène.

**3.** Procédé selon la revendication 1 ou la revendication 2, dans lequel les métaux du catalyseur sont présents sous forme d'oxyde ou de sulfure.

**4.** Procédé selon l'une quelconque des revendications 1 à 3, dans lequel le catalyseur est supporté sur un support choisi parmi alumine, silice, dioxyde de titane, zéolites, charbon, zircone, magnésie (MgO), des argiles, des hydrotalcites et autres, ainsi que les mélanges de deux ou plusieurs d'entre eux.

**5.** Procédé selon l'une quelconque des revendications 1 à 4, comprenant au moins les étapes suivantes :

a) mise en contact du disulfure de carbone avec de l'hydrogène, en présence d'au moins un catalyseur selon l'une quelconque des revendications 1 à 4, ayant éventuellement subi un pré-traitement de conversion des oxydes métalliques en sulfures métalliques ;
b) conduite de la réaction sous pression d'hydrogène à une température comprise entre 100°C et 400°C, de préférence comprise entre 200°C et 300°C ; et
c) récupération du produit d'hydrogénation, le méthylmercaptan, avec séparation du $H_2S$ formé.

**6.** Procédé selon la revendication 1, dans lequel le ratio $H_2/CS_2$ est compris entre 2:1 et 9:1, avantageusement compris entre 3:1 et 6:1.

7. Procédé selon la revendication 5, **caractérisé par le fait qu'**il est mis en œuvre en continu.

8. Procédé selon la revendication 5, **caractérisé par le fait qu'**il est mis en œuvre sans solvant, sans eau, éventuellement en présence d'un gaz inerte, tel que azote, argon, et autres.


**Patentansprüche**

1. Verfahren zur katalytischen Hydrierung, wobei es sich um eine Reaktion zur katalytischen Hydrierung von Kohlendisulfid ($CS_2$) zu Methylmercaptan handelt, bei dem es sich bei dem Hydrierkatalysator um einen Katalysator handelt, der die Kombinationen von Metallen Co/Mo, Ni/Mo, Ni/W oder W/Mo umfasst; wobei die Metalle mit Kaliumhydroxid oder Kaliumoxid dotiert sind und die Menge an Kaliumhydroxid bzw. Kaliumoxid, bezogen auf das Gesamtgewicht des Katalysators, zwischen 8 und 14 Gew.-% liegt; und bei dem das $H_2/CS_2$-Molverhältnis zwischen 1:1 und 10:1 liegt.

2. Verfahren nach Anspruch 1, bei dem es sich bei der Kombination von zwei Metallen um eine Kombination von Nickel und Molybdän handelt.

3. Verfahren nach Anspruch 1 oder Anspruch 2, wobei die Metalle des Katalysators in Oxid- oder Sulfidform vorliegen.

4. Verfahren nach einem der Ansprüche 1 bis 3, wobei der Katalysator auf einem Träger, der aus Aluminiumoxid, Siliciumdioxid, Titandioxid, Zeolithen, Kohle, Zirconiumdioxid, Magnesiumoxid (MgO), Tonen, Hydrotalciten und anderen sowie Gemischen von zwei oder mehr davon ausgewählt ist, geträgert ist.

5. Verfahren nach einem der Ansprüche 1 bis 4, das mindestens die folgenden Schritte umfasst:

   a) Inkontaktbringen von Kohlendisulfid mit Wasserstoff in Gegenwart mindestens eines Katalysators nach einem der Ansprüche 1 bis 4, der gegebenenfalls einer Vorbehandlung zur Umwandlung der Metalloxide in Metallsulfide unterworfen wurde;
   b) Durchführen der Reaktion unter Wasserstoffdruck bei einer Temperatur zwischen 100 °C und 400 °C, vorzugsweise zwischen 200 °C und 300 °C; und
   c) Gewinnen des Hydrierungsprodukts, Methylmercaptan, mit Abtrennung des gebildeten $H_2S$.

6. Verfahren nach Anspruch 1, bei dem das $H_2/CS_2$-Verhältnis zwischen 2:1 und 9:1, vorteilhafterweise zwischen 3:1 und 6:1, liegt.

7. Verfahren nach Anspruch 5, **dadurch gekennzeichnet, dass** es kontinuierlich durchgeführt wird.

8. Verfahren nach Anspruch 5, **dadurch gekennzeichnet, dass** es ohne Lösungsmittel, ohne Wasser und gegebenenfalls in Gegenwart eines Inertgases, wie Stickstoff, Argon und anderen, durchgeführt wird.


**Claims**

1. Catalytic hydrogenation process which is a catalytic hydrogenation reaction of carbon disulfide ($CS_2$) to methyl mercaptan, wherein the hydrogenation catalyst is a catalyst comprising the metal combinations Co/Mo, Ni/Mo, Ni/W or W/Mo;

   said metals being doped with potassium hydroxide or potassium oxide, and the amount of potassium hydroxide or potassium oxide relative to the total weight of the catalyst being between 8% and 14% by weight; and wherein the molar $H_2/CS_2$ ratio is between 1:1 and 10:1.

2. Process according to Claim 1, wherein the combination of two metals is a combination of nickel and molybdenum.

3. Process according to Claim 1 or Claim 2, wherein the metals of the catalyst are present in oxide or sulfide form.

4. Process according to any one of Claims 1 to 3, wherein the catalyst is supported on a support chosen from alumina, silica, titanium dioxide, zeolites, charcoal, zirconia, magnesia (MgO), clays, hydrotalcites and others, and the mixtures of two or more of them.

5. Process according to any one of Claims 1 to 4, comprising at least the following steps:

   a) bringing carbon disulfide into contact with hydrogen in the presence of at least one catalyst according to any one of Claims 1 to 4 which has optionally been subjected to a pre-treatment for conversion of the metal oxides to metal sulfides;
   b) carrying out the reaction under hydrogen pressure at a temperature of between 100°C and 400°C, preferably of between 200°C and 300°C; and
   c) recovering the hydrogenation product, methyl mercaptan, with separation of the $H_2S$ formed.

6. Process according to Claim 1, wherein the $H_2/CS_2$ ratio is between 2:1 and 9:1, advantageously between 3:1 and 6:1.

7. Process according to Claim 5, **characterized in that** it is carried out continuously.

8. Process according to Claim 5, **characterized in that** it is carried out without solvent and without water and optionally in the presence of an inert gas, such as nitrogen, argon and others.

**EP 2 349 558 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3488739 A
- US 3880933 A
- US 4543434 A
- US 4822938 A
- US 4864074 A
- WO 2004043883 A